(19) Europäisches Patentamt
European Patent Office
Office européen des brevets

(11) Veröffentlichungsnummer: **0 075 773**
**B1**

(12) # EUROPÄISCHE PATENTSCHRIFT

(45) Veröffentlichungstag der Patentschrift: **06.11.85**

(51) Int. Cl.⁴: **C 09 B 44/08,** D 21 H 3/80, D 06 P 1/08

(21) Anmeldenummer: **82108394.6**

(22) Anmeldetag: **11.09.82**

(54) **Farbstoffe, ihre Herstellung und Verwendung.**

(30) Priorität: **25.09.81 DE 3138182**

(43) Veröffentlichungstag der Anmeldung: **06.04.83 Patentblatt 83/14**

(45) Bekanntmachung des Hinweises auf die Patenterteilung: **06.11.85 Patentblatt 85/45**

(84) Benannte Vertragsstaaten: **CH DE FR GB LI**

(56) Entgegenhaltungen: **GB - A - 2 076 421**

(73) Patentinhaber: **BAYER AG, Konzernverwaltung RP Patentabteilung, D-5090 Leverkusen 1 Bayerwerk (DE)**

(72) Erfinder: **Kunde, Klaus, Dr., Uppersberg 45, D-5090 Leverkusen 1 (DE)**
Erfinder: **Wild, Peter, Dr., Hainstrasse 7, D-6305 Alten Buseck (DE)**

EP 0 075 773 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Gegenstand der Erfindung sind substantive Farbstoffe der allgemeinen Formel

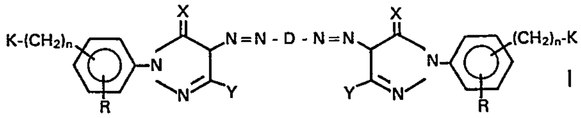
I

worin
X O oder NH,
D der Rest einer Bisdiazokomponente,
Y $C_1$-$C_4$-Alkyl oder $C_1$-$C_4$-Alkoxycarbonyl,
R Wasserstoff oder ein nichtionischer Substituent,
n 0, 1 oder 2,
K ein Rest der Formel

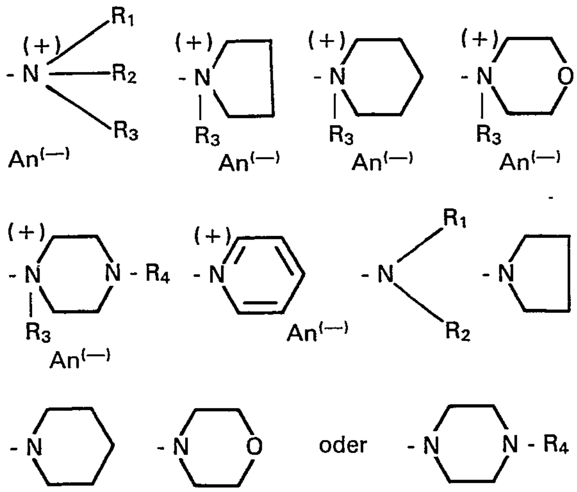
oder

$R_1$, $R_2$, $R_3$ und $R_4$ unabhängig voneinander Wasserstoff, $C_1$-$C_4$-Alkyl, $C_2$-$C_4$-β-hydroxyalkyl oder Benzyl und
$An^{(-)}$ ein Anion sind,
ein Verfahren zu ihrer Herstellung und ihre Verwendung zum Färben von natürlichen und synthetischen Substraten und Massen, insbesondere Papier.

D steht vorzugsweise für den Rest einer Bisdiazokomponente der Benzolreihe, insbesondere für einen Rest der Formel

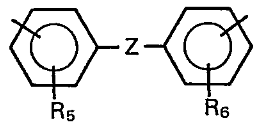

worin
Z eine direkte Bindung oder ein Brückenglied und
$R_5$ und $R_6$ unabhängig voneinander Wasserstoff oder einen nichtionischen Substituenten darstellen.

Unter einem nichtionischen Substituenten R, $R_5$ und $R_6$ wird vorzugsweise Halogen, z.B. Fluor, Chlor oder Brom, $C_1$-$C_4$-Alkyl und $C_1$-$C_4$-Alkoxy verstanden.

Als Anionen kommen übliche farblose organische und anorganische Anionen, beispielsweise Chlorid, Bromid, Jodid, Hydroxyl, Hydrogensulfat, Sulfat, Nitrat, Dihydrogenphosphat, Hydrogenphosphat, Phosphat, Carbonat, Methosulfat, Ethosulfat, Formiat, Acetat, Propionat, Benzolsulfonat und Toluolsulfonat in Betracht.

Das Anion ist im allgemeinen durch das Herstellungsverfahren gegeben. Die Anionen können in bekannter Weise gegen andere Anionen ausgetauscht werden. Vorzugsweise liegen die Chloride, Hydrogensulfate, Sulfate, Methosulfate, Phosphate, Formiate, Acetate oder Hydroxide vor.

Vorteilhaft sind Farbstoffe der allgemeinen Formel

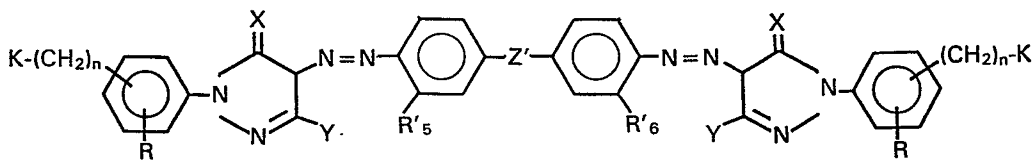
(II)

worin
Z' eine direkte Bindung oder ein Brückenglied der Formel -CONH-, -CO-, -NH-CO-NH-, $-CH_2-CH_2-$, $-CH_2-$, -CH=CH-, O, S oder NH und
R', $R'_5$ und $R'_6$ unabhängig voneinander Wasserstoff, Methyl, Chlor, Brom, Methoxy, Ethoxy sind, und
worin X, Y, n und K die in Formel I angegebene Bedeutung haben.

Zur Herstellung der Farbstoffe (I) werden Verbindungen der allgemeinen Formel

$$H_2N\text{-}D\text{-}NH_2 \qquad \text{(III)}$$

in an sich bekannter Weise bisdiazotiert und mit Verbindungen der allgemeinen Formel

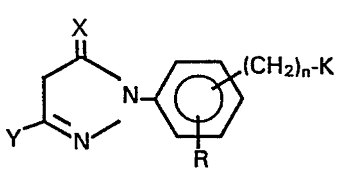
(IV)

worin D, X, Y, R, n und K die in Formel (I) angegebene Bedeutung besitzen, gekuppelt.

Gegenstand der Erfindung sind ebenfalls Verbindungen der allgemeinen Formel

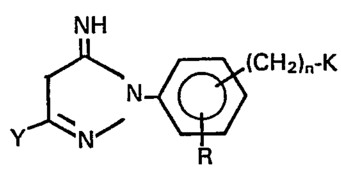
(V)

worin Y, R, n und K die in Formel (I) angegebene Bedeutung haben, und ein Verfahren zu ihrer Herstellung, das darin besteht, dass man Anilinderivate der allgemeinen Formel

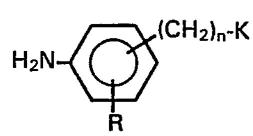
(VI)

in an sich bekannter Weise diazotiert, die Diazoniumverbindungen zu den Hydrazinen reduziert und diese mit Verbindungen der allgemeinen Formel

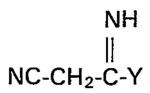

(VII)

worin R, n, K und Y die in Formel (I) angegebene Bedeutung besitzen, kondensiert und schliesslich den Pyrazolring schliesst.

Als Anilinderivate der allgemeinen Formel (VI) kommen z.B. folgende in Betracht:

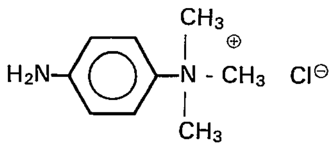

a

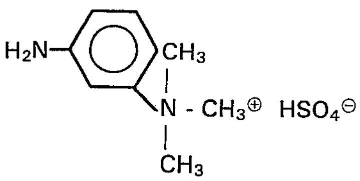

b

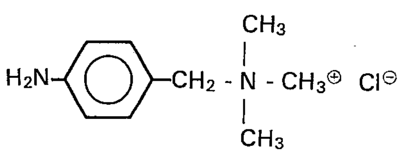

c

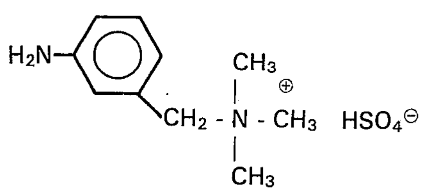

d

Als Anionen können auch die Anionen anderer anorganischer oder organischer Säuren verwendet werden, z.B. Bromid, Methosulfat, Benzolsulfonat, Acetat.

Als Verbindungen der allgemeinen Formel (VII) können z.B. Cyanaceton, Cyanacetonamid, Cyanbrenztraubensäuremethylester eingesetzt werden, die zweckmässigerweise in Form ihrer Natrium-Salze zur Umsetzung gebracht werden.

Die Diazotierung der Anilinderivate erfolgt wie üblich bei Temperaturen zwischen 0 und 20°C. Die Reduktion zu den entsprechenden Hydrazinen kann man z.B. mit schwefliger Säure bei pH-Werten zwischen 4 und 7 durchführen, bei Temperaturen zwischen 20 und 50°. Diese Hydrazine kondensiert man mit Verbindungen der allgemeinen Formel (VII) bei pH-Werten zwischen 0 und 2 bei Temperaturen zwischen 60 und 100°C, wobei auch der Pyrazolring geschlossen wird. Die erfindungsgemässen Verbindungen werden vorzugsweise ohne Zwischenisolierung weiterverarbeitet.

Als Diamine der allgemeinen Formel (III) kommen z.B. in Betracht:

4,4'-Diaminobenzanilid
4,4'-Diaminobenzophenon
4,4'-Diamino-N,N'-diphenylharnstoff
1,2-Bis-(4'-Aminophenyl)-ethan
1,2-Bis-(4'-Aminophenyl)-methan
3,3'-Dianisidin
4,4'-Diaminodiphenylamin
4,4'-Diaminodiphenylether
4,4'-Diaminostilben.

Als Kupplungskomponenten der allgemeinen Formel (IV) können z.B. verwendet werden:

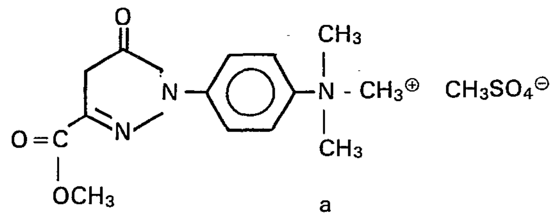

a

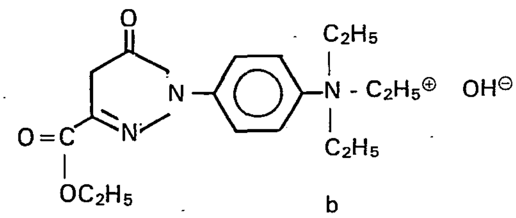

b

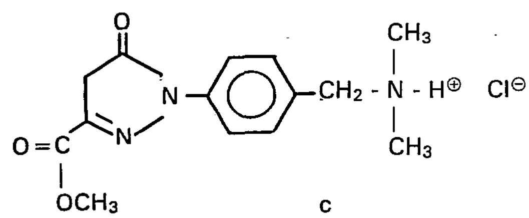

c

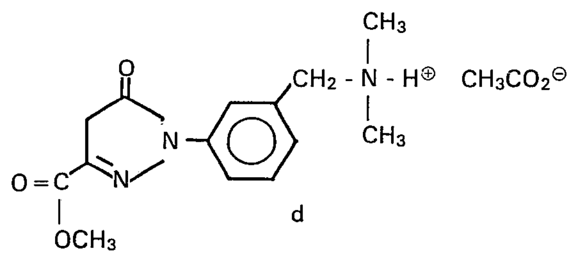

d

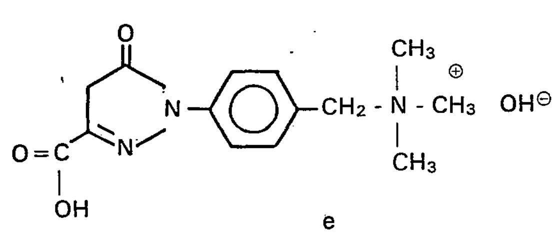

e

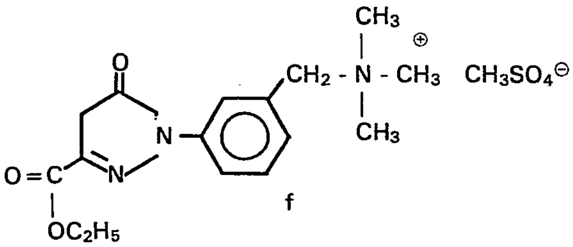

f

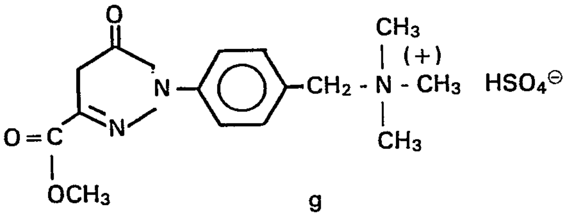

g

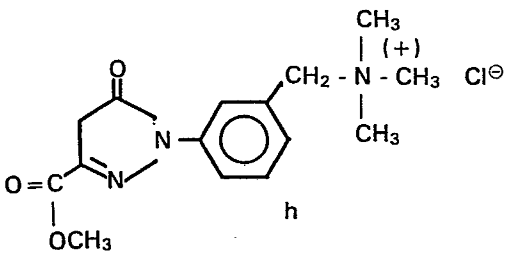

h

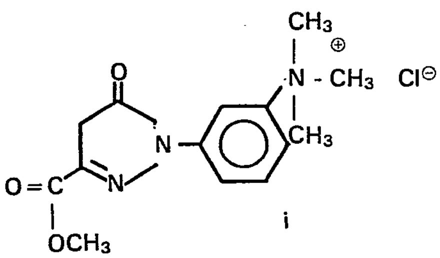

i

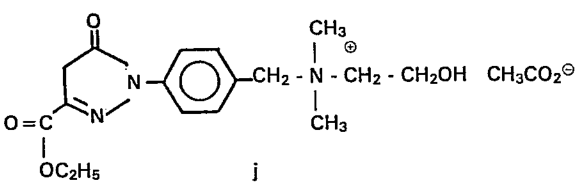

j

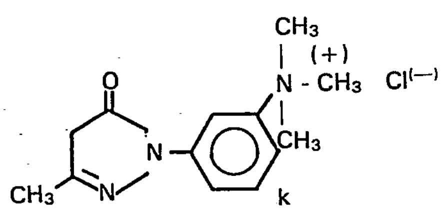

k

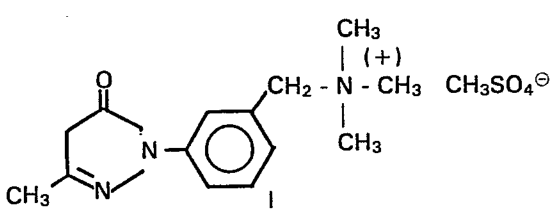

l

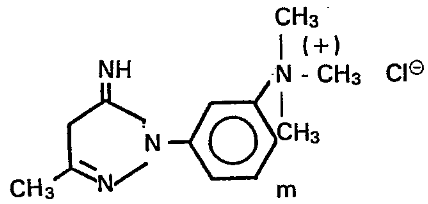

m

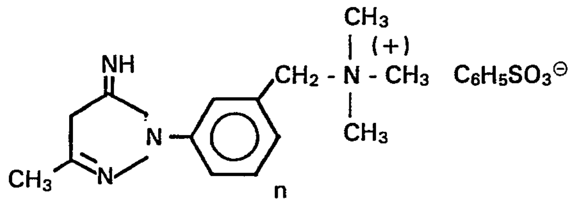

n

Als Anionen kommen in den o.a. Verbindungen jeweils auch andere anorganische oder organische Anionen in Frage, also z.B. jeweils auch Chlorid, Bromid, Acetat, Methosulfat, Benzolsulfonat oder Hydroxid.

Die Kupplung erfolgt üblicherweise bei Temperaturen zwischen 0 und 50°C, vorzugsweise bei Temperaturen zwischen 0 und 30°C bei pH-Werten zwischen 2 und 9, vorzugsweise zwischen 3 und 5. Die Farbstoffe können isoliert und getrocknet werden, sie können aber auch mit geeigneten Lösungsmitteln in stabile, konzentrierte Lösungen überführt werden.

Solche Lösungsmittel sind z.B. wässrige Lösungen von Mineralsäuren und/oder organischen Säuren, z.B. Salzsäure, Schwefelsäure, Phosphorsäure, Ameisensäure, Essigsäure, Glykolsäure, Milchsäure, Methansulfonsäure.

Als Lösungsmittel können aber auch organische Lösungsmittel wie z.B. Ethylenglykol, Diglykol, Triglykol, Glycerin, Ethylglykolmonomethylether, Diglykolmonoethylether, Diglykolmonobutylether u.ä. verwendet oder mit verwendet werden.

Die erfindungsgemässen Farbstoffe, welche basische Aminogruppen enthalten, werden vorzugsweise vor ihrer Verwendung zum Färben durch Zugabe einer organischen oder anorganischen Säure in ihre entsprechenden Ammonium-Verbindungen überführt.

Die erfindungsgemässen Farbstoffe färben kationisch anfärbbare Materialien wie z.B. Polyacrylnitril, sauer modifizierten Polyester und Polyamid, Wolle und Leder, aber auch cellulosehaltige Materialien wie z.B. Baumwolle und Celluloseregenerat-Fasern, insbesondere Papier mit guten Echtheiten in orangefarbenen bis gelben Tönen.

Die Farbstoffe können zum Papiermasse-Färben oder zur Papieroberflächenfärbung eingesetzt werden. Die sind geeignet für geleimte und für ungeleimte Papiersorten ausgehend von gebleichtem oder ungebleichtem Zellstoff verschiedener Provenienz wie Nadel- oder Laubholz-Sulfit- und/oder -Sulfat-Zellstoff.

Das Färben erfolgt vorzugsweise bei pH-Werten von 4 bis 8, insbesondere pH 5 bis 7. Die Färbetemperatur beträgt im allgemeinen 10° bis 50°C, vorzugsweise etwa 20°.

Die bei der Papierfärbung und -Herstellung üblichen Hilfsmittel und Füllstoffe können beim Einsatz

der erfindungsgemässen Farbstoffe mitverwendet werden. Die Farbstoffe besitzen bei der Papierfärbung ein ausgezeichnetes Ziehvermögen.

Die mit den erfindungsgemässen Farbstoffen erhaltenen Papierfärbungen zeichnen sich durch sehr gute Wasserechtheit (Ausblutechtheit) sowie Säure-, Alkali- und Alaun-Echtheit aus. Hervorzuheben ist aber die überraschend hohe Lichtechtheit der Papierfärbungen. Auch auf die Brillanz und Klarheit der Farbtöne ist hinzuweisen. Ferner ist das Kombinationsverhalten mit geeigneten Farbstoffen sehr gut.

Die Farbstoffe können auch zum Färben von Baumwolle und ohne Celluloseregenerat-Fasern eingesetzt werden, ohne dass die bisher üblichen Hilfsmittel wie z.B. die Tannin-Vorbehandlung angewendet werden müssen; auch auf die Zugabe von Salz kann verzichtet werden.

Das Färben erfolgt dann vorzugsweise bei Werten von 4 bis 8, insbesondere bei pH 5 bis 7. Die Färbetemperatur beträgt im allgemeinen 60° bis 130°C, vorzugsweise 80° bis 100°C.

*Beispiel A*

36 g 3-Aminophenyltrimethylammoniumhydrogensulfat werden in 200 ml Wasser gelöst und nach Zugabe von 40 ml 30%iger Salzsäure und 100 g Eis mit 10 g Natriumnitrit diazotiert. 80 ml 40% Bisulfit-Lösung werden mit Natronlauge auf einen pH-Wert von 6 gebracht, dann gibt man bei 30° die Lösung der Diazoniumverbindung zu. Man rührt ca. 1 Stunde. Wenn keine Diazoniumverbindung mehr nachweisbar ist, werden 60 ml 30%ige Salzsäure zugesetzt. Man erhitzt auf 95°C und hält die Temperatur, bis alles $SO_2$ ausgetrieben ist. Nun gibt man bei pH = 1 eine Lösung von 15,1 g des Natriumsalzes von Cyanceton zu und rührt noch 2 Stunden bei dieser Temperatur. Man erhält eine Lösung der Verbindung der Formel

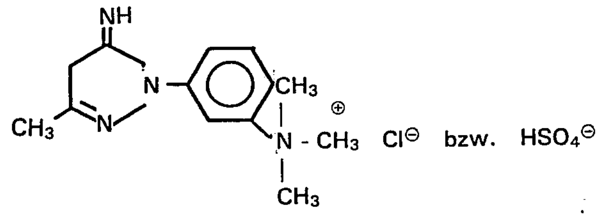

Die Ausbeute beträgt 75% (Kupplungswert).

*Beispiel 1*

11,3 g 4,4'-Diaminobenzanilid werden in 200 ml Wasser mit 50 g Eis und 300 ml 30%iger Salzsäure verrührt und mit 6,9 g Natriumnitrit diazotiert. Man fügt eine Lösung von 31,2 g der Kupplungskomponente i in 200 ml Wasser zu und bringt den pH-Wert mit 20%iger Natriumacetat-Lösung auf 3,5-4. Man rührt noch 2 Stunden zur Vervollständigung der Kupplung. Der Farbstoff ist ausgefallen. Er wird isoliert und in 700 ml 50%iger Essigsäure gelöst. Man erhält eine stabile Lösung des Farbstoffes der Formel

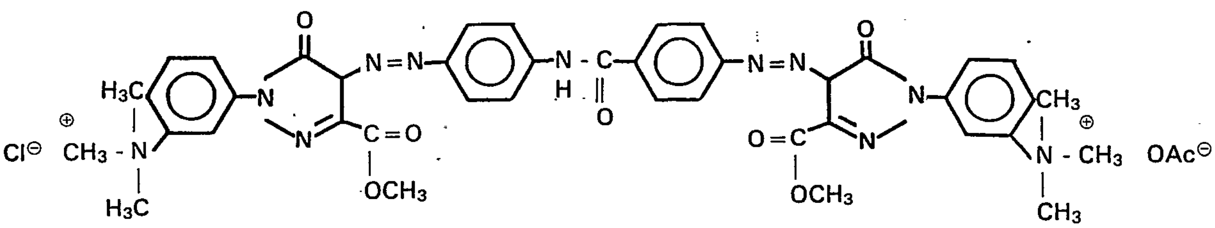

der Papier in der Masse in orangefarbenen Tönen anfärbt.

*Beispiel 2-6*

Verwendet man statt der in Beispiel 1 eingesetzten Kupplungskomponente äquimolare Mengen der Kupplungskomponenten a, c, d, g oder h, so erhält man ebenfalls Lösungen der entsprechenden orangefarbenen Farbstoffe.

*Beispiel 7 und 8*

Verwendet man statt der in Beispiel 1 eingesetzten Kupplungskomponente äquimolare Mengen der Kupplungskomponenten k und m, so erhält man ebenfalls Lösungen der entsprechenden gelben bzw. grünstichig gelben Farbstoffe.

*Beispiel 9*

Ein aus 60% Holzschliff und 40% ungebleichtem Sulfitzellstoff bestehender Trockenstoff wird im Holländer mit Wasser angeschlagen und bis zum Mahlgrad 40° SR gemahlen, so dass der Trockengehalt etwas über 2,5% liegt und anschliessend mit Wasser auf 2,5% Trockengehalt des Dickstoffs eingestellt.

200 Teile dieses Dickstoffs werden mit 5 Teilen einer 0,5%igen essigsauren wässrigen Lösung des Farbstoffs aus Beispiel 1 versetzt, ca. 5 Minuten verrührt, mit 2% Harzleim und 3% Alaun (bezogen auf Trockenstoff) versetzt und wieder einige Minuten bis zur Homogenität verrührt. Man verdünnt die Masse nur mit ca. 500 Teilen Wasser und stellt hieraus in üblicher Weise durch Absaugen über einen Blattbildner Papierblätter her. Die Papierblätter weisen eine orangefarbene Färbung auf. Das Abwasser der Färbung ist praktisch farbstofffrei.

*Beispiel 10*

Verwendet man statt des in Beispiel 9 eingesetzten Farbstoffs den Farbstoff aus Beispiel 2, so erhält man ebenfalls orange gefärbte Papierblätter und ein praktisch farbstoffreines Abwasser.

*Beispiel 11 und 12*

Auch beim Färben von ungebleichter Papiermasse unter sonst gleichen Färbebedingungen und bei der Verwendung von gebleichtem Sulfitzellstoff erhält man orangefarbene Papierfärbungen und praktisch farbstofffreie Abwässer.

**Patentansprüche**

1. Farbstoffe der allgemeinen Formel

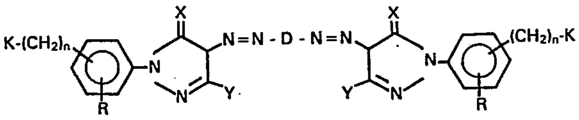

worin
X O oder NH,
D der Rest einer Bisdiazokomponente,
Y $C_1$-$C_4$-Alkyl oder $C_1$-$C_4$-Alkoxycarbonyl,
R Wasserstoff oder ein nichtionischer Substituent,
n 0, 1 oder 2,
K ein Rest der Formel

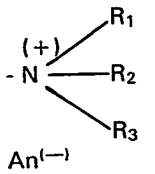
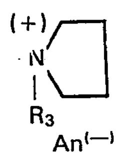
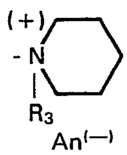
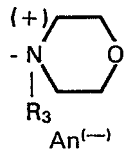
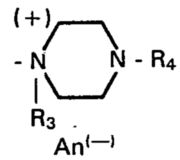
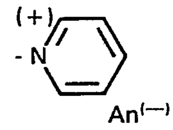
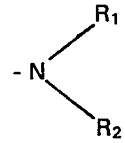
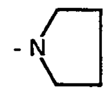
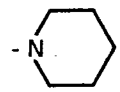
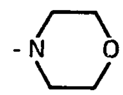
oder
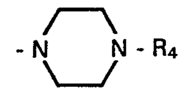

$R_1$, $R_2$, $R_3$ und $R_4$ unabhängig voneinander Wasserstoff, $C_1$-$C_4$-Alkyl, $C_2$-$C_4$-β-Hydroxyalkyl oder Benzyl und
$An^{(-)}$ ein Anion sind.

2. Farbstoffe gemäss Anspruch 1 der allgemeinen Formel

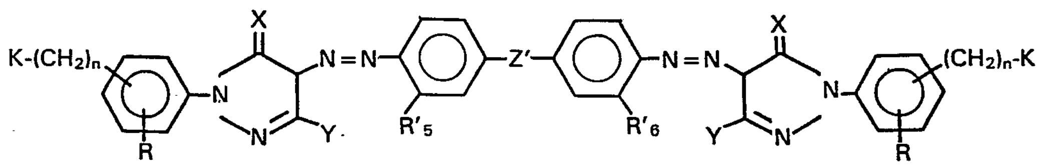

worin
Z′ eine direkte Bindung oder ein Brückenglied der Formel -CONH-, -CO-, -NH-, CO-NH-, $-CH_2-CH_2-$, $-CH_2-$, -CH=CH-, O, S oder NH und
R′, $R'_5$ und $R'_6$ unabhängig voneinander Wasserstoff, Methyl, Chlor, Brom, Methoxy, Ethoxy sind, und
worin X, Y, n und K die in Anspruch 1 angegebene Bedeutung haben.

3. Verfahren zur Herstellung von kationischen Farbstoffen des Anspruchs 1, dadurch gekennzeichnet, dass man Verbindungen der allgemeinen Formel

$$H_2N - D - NH_2$$

worin D die in Anspruch 1 angegebene Bedeutung hat, bisdiazotiert und mit Verbindungen der allgemeinen Formel

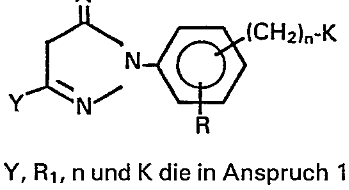

worin X, Y, $R_1$, n und K die in Anspruch 1 angegebene Bedeutung haben, kuppelt.

4. Verfahren zum Färben von natürlichen und synthetischen Substraten und Massen, dadurch gekennzeichnet, dass man Farbstoffe des Anspruchs 1 verwendet.

5. Verfahren zum Färben von Papier, dadurch gekennzeichnet, dass man Farbstoffe des Anspruchs 1 verwendet.

6. Papier, gefärbt mit den Farbstoffen des Anspruchs 1.

7. Verbindungen der allgemeinen Formel

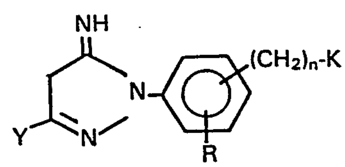

worin Y, R, n und K die in Anspruch 1 angegebene Bedeutung haben.

8. Verfahren zur Herstellung von Verbindungen des Anspruchs 7, dadurch gekennzeichnet, dass man Anilinderivate der allgemeinen Formel

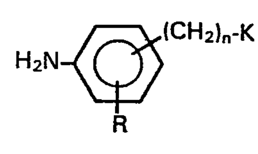

diazotiert, die Diazoniumverbindungen zu den Hydrazinen reduziert und diese mit Verbindungen der allgemeinen Formel

NH
||
$NC - CH_2$ C - Y

worin R, n, K und Y die in Anspruch 1 angegebene Bedeutung besitzen, kondensiert und schliesslich den Pyrazolring schliesst.

## Revendications

1. Colorant de formule générale

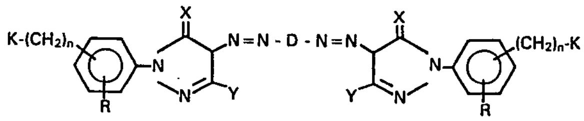

dans laquelle
X est O ou NH,
D est le reste d'un composant bis-diazoïque,
Y est un reste alkyle en $C_1$-$C_4$ ou (alcoxy en $C_1$-$C_4$)carbonyle,
R est l'hydrogène ou un substituant non ionique,
n est égal à 0, 1 ou 2,
K est un reste de formule

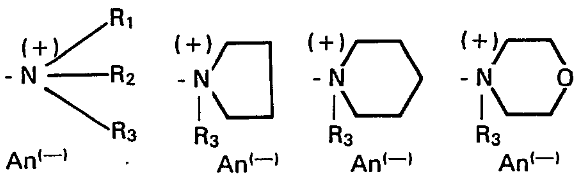

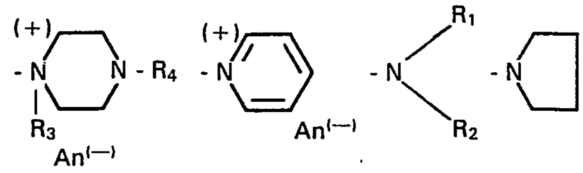

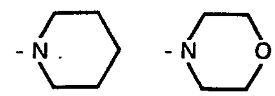

ou

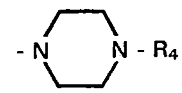

$R_1$, $R_2$, $R_3$ et $R_4$ sont chacun indépendamment l'un de l'autre l'hydrogène ou un groupe alkyle en $C_1$-$C_4$, β-hydroxyalkyle en $C_2$-$C_4$ ou benzyle, et $An^{(-)}$ est un anion.

2. Colorants selon la revendication 1, de formule générale

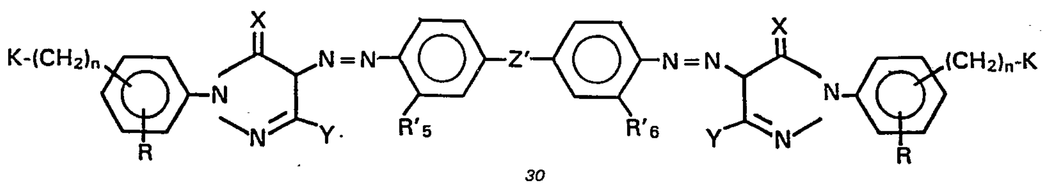

dans laquelle
Z' est une liaison directe ou un groupement de pontage de formule -CONH-, -CO-, -NH-CO-NH-, $-CH_2-CH_2-$, $-CH_2-$, -CH=CH-, O, S ou NH et
R', $R'_5$ et $R'_6$ sont indépendamment l'un de l'autre l'hydrogène, le chlore, le brome ou un groupe méthyle, méthoxy ou éthoxy, et
X, Y, n et K ont la signification indiquée à la revendication 1.

3. Procédé pour la fabrication des colorants cationiques de la revendication 1, caractérisé en ce que l'on soumet des composés de formule générale

$$H_2N - D - NH_2$$

dans laquelle D a la signification indiquée à la revendication 1, à la bis-diazotation et on copule avec des composés de formule générale

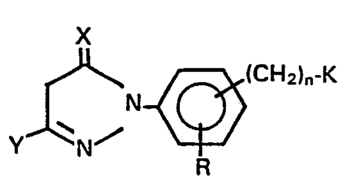

dans laquelle X, Y, $R_1$, n et K ont la signification indiquée dans la revendication 1.

4. Procédé pour la teinture de substrats et masses naturels et synthétiques, caractérisé en ce que l'on utilise des colorants selon la revendication 1.

5. Procédé pour la teinture du papier, caractérisé en ce que l'on utilise des colorants selon la revendication 1.

6. Papier, teint avec les colorants selon la revendication 1.

7. Composés de formule générale

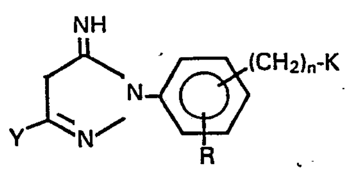

dans laquelle Y, R, n et K ont la signification indiquée à la revendication 1.

8. Procédé pour la fabrication de composés selon la revendication 7, caractérisé en ce que l'on diazote des dérivés d'aniline de formule générale

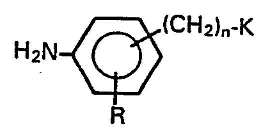

on réduit les composés diazonium et les hydrazines et on condense celles-ci avec des composés de formule générale

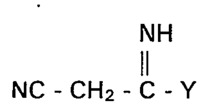

où R, n, K et Y ont la signification indiquée à la revendication 1, et enfin on ferme le cycle pyrazole.

**Claims**

1. Dyestuffs of the general formula

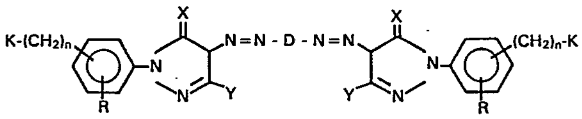

wherein
X is O or NH
D is the radical of a bisdiazo component,
Y is $C_1$-$C_4$-alkyl or $C_1$-$C_4$-alkoxycarbonyl,
R is hydrogen or a non-ionic substituent,
n is 0, 1 or 2,
K is a radical of the formula

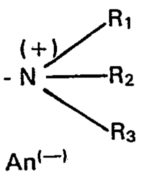
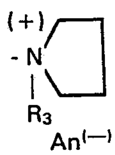
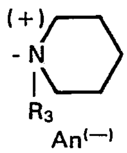
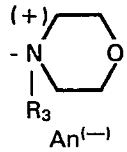
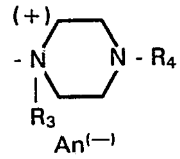
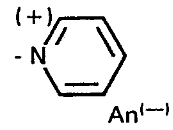
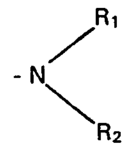
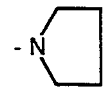
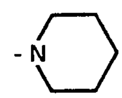
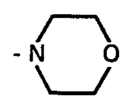

or

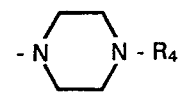

$R_1$, $R_2$, $R_3$ and $R_4$ independently of one another are hydrogen, $C_1$-$C_4$-alkyl, $C_2$-$C_4$-β-hydroxyalkyl or benzyl and
$An^{(-)}$ is an anion.

2. Dyestuffs according to claim 1 of the general formula

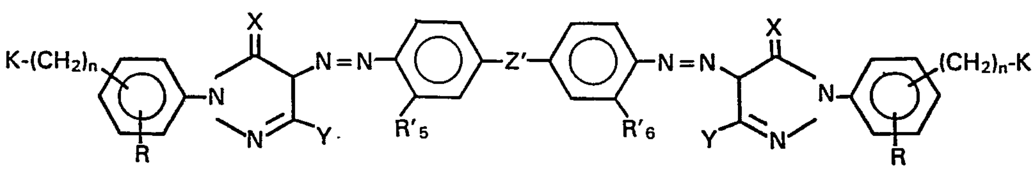

wherein
Z' is a direct bond or a bridge member of the formula -CONH-, -CO-, -NH-CO-NH-, $-CH_2-CH_2-$, $-CH_2-$, -CH=CH-, O, S or NH and
R', $R'_5$ and $R'_6$ independently of one another are hydrogen, methyl, chlorine, bromine, methoxy or ethoxy and
wherein
X, Y, n and K have the meaning indicated in claim 1.

3. Process for preparing cationic dyestuffs of claim 1, characterised in that compounds of the general formula

$$H_2N - D - NH_2$$

wherein
D has the meaning indicated in claim 1 are bis-diazotised, and the product is coupled with compounds of the general formula

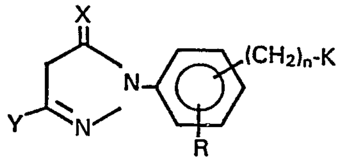

wherein
X, Y, $R_1$, n and K have the meaning indicated in claim 1.

4. Process for dyeing/colouring natural and synthetic substrates and compositions, characterised in that dyestuffs of claim 1 are used.

5. Process for colouring paper, characterised in that dyestuffs of claim 1 are used.

6. Paper, coloured with the dyestuffs of claim 1.

7. Compounds of the general formula

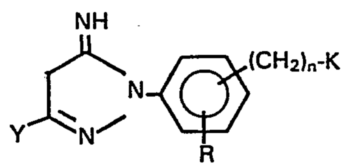

wherein
Y, R, n and K have the meaning indicated in claim 1.

8. Process for the preparation of compounds of claim 7, characterised in that aniline derivatives of the general formula

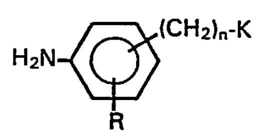

are diazotised, the diazonium compounds are reduced to the hydrazines and the latter are condensed with compounds of the general formula

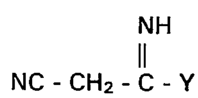

wherein
R, n, K and Y have the meaning indicated in claim 1 and finally the pyrazole ring is closed.